# EUROPEAN PATENT APPLICATION

(11) **EP 3 628 658 A1**
(43) Date of publication of application: **01.04.2020**
(21) Application number: 19199427.6
(22) Date of filing: 25.09.2019
(51) Int. Cl.: C07C 221/00, C07C 225/14, C07D 207/08

(54) **SYNTHESIS OF SUBSTITUTED ENONES**

(30) Priority: 28.09.2018 DE 102018124026
(71) Applicant: Henkel AG & Co. KGaA, 40589 Düsseldorf (DE)
(72) Inventor: SCHAEFER, Sascha, 40822 Mettmann (DE); SCHMIDT, Nora, 63452 Hanau (DE); NEUHAUS, Svenja, 47179 Duisburg (DE)
(74) Representative: Viering, Jentschura & Partner mbB Patent- und Rechtsanwälte

(57) **Abstract**

The present invention is in the field of basic and fine chemical synthesis and washing or cleaning agents and relates to a method for preparing an enone compound of formula (I) by reacting a ketone compound of formula (II) with an amine compound of formula (III) in the presence of at least one oxidant. Furthermore, the invention refers to the enone compound of formula (I) obtained by the method according to the invention and to a washing or cleaning agent comprising the enone compound of formula (I) obtained by the method according to the invention. In addition, use of the enone compound of formula (I) as fine chemical, basic chemical, insect repellant, pharmaceutical compound, fragrance or pro-fragrance is part of the invention.

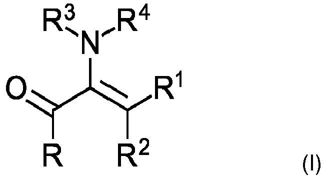

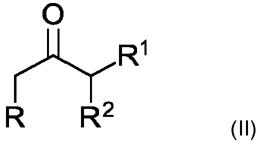

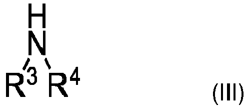

## Description

The present invention is in the field of basic and fine chemical synthesis and washing or cleaning agents and relates to a method for preparing an enone compound of formula (I). Furthermore, the invention refers to the enone compound of formula (I) obtained by the method according to the invention and to a washing or cleaning agent comprising the enone compound of formula (I) obtained by the method according to the invention. In addition, use of the enone compound of formula (I) as fine chemical, basic chemical, insect repellant, pharmaceutical compound, fragrance or pro-fragrance is part of the invention.

Enones, especially alpha substituted enones, are valuable building blocks for fine and basic chemicals, which are applied in a wide field ranging from pharmaceuticals, fragrances, perfumes, performance polymers, ingredients for washing or cleaning compositions and many others. In particular, enones with a nitrogen bearing substituent in alpha-position are difficult to synthesize. Often, they are only obtainable by complex and multi-step synthesis methods.

Therefore, there is a need for new and simple synthesis methods to produce valuable enones with nitrogen bearing substituents in alpha-position. Especially, a low number of synthesis steps and high atom-economic use of starting materials are desired.

Surprisingly, the inventors of the present invention found that alpha-substituted enones can be produced by direct synthesis of simple amines and ketones in the presence of an oxidant, preferably in the presence of a Brønsted acid or Lewis acid, more preferably at elevated temperatures.

Therefore, in a first aspect, the invention relates to a method for preparing an enone compound of formula (I) 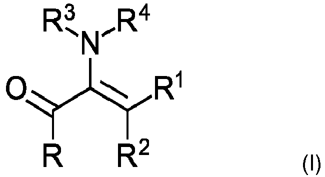 by reacting a ketone compound of formula (II) 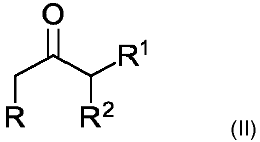 with an amine compound of formula (III) 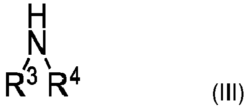 in the presence of at least one oxidant, preferably at a temperature in the range of 0 to 300 °C, more preferably in the range of 30 to 100 °C; wherein
(1) R, R¹, R², R³ and R⁴ are independently of each other selected from hydrogen, substituted or unsubstituted linear C1-C20 alkyl groups or branched C3-C20 alkyl groups; substituted or unsubstituted linear C2-C20 alkenyl groups or branched C3-C20 alkenyl groups; substituted or unsubstituted linear C2-C20 alkynyl groups or branched C4-C20 alkynyl groups; substituted or unsubstituted C3-C10 cycloalkyl, C3-C10 cycloalkenyl or aryl groups; substituted or unsubstituted, linear C4-C20 alkylcycloalkyl, C4-C20 alkylcycloalkenyl, C4-C20 alkenylcycloalkyl, C4-C20 alkenylcycloalkenyl, C5-C20 alkynylcycloalkyl or C5-C20 alkynylcycloalkenyl groups, or branched C5-C20 alkylcycloalkyl, C5-C20 alkylcycloalkenyl, C5-C20 alkenylcycloalkyl, C5-C20 alkenylcycloalkenyl, C6-C20 alkynylcycloalkyl or C6-C20 alkynylcycloalkenyl groups, or substituted or unsubstituted linear C7-C20 alkylaryl, C8-C20 alkenylaryl or C8-C20 alkynylaryl groups, or branched C8-C20 alkylaryl, C8-C20 alkenylaryl or C9-C20 alkynylaryl groups; or substituted or unsubstituted linear C1-C20 alkylester, C1-C20 alkylether, or C1-C20 alkylmethoxy, or branched C2-C20 alkylester, C2-C20 alkylether, or C2-C20 alkylmethoxy groups; wherein each of the groups of R, R¹, R², R³ and R⁴ may further contain 1 to 6 heteroatoms, or wherein
(2) R and R² and/or R³ and R⁴ each form a substituted or unsubstituted hydrocarbon ring with 3 to 12 carbon atoms, wherein each of the hydrocarbon rings may further contain at least one heteroatom, selected from O, N, S, and P, and/or wherein each of the hydrocarbon rings may further contain at least one double bond; and
   wherein the remaining groups are as defined above.

In a second aspect, the invention claims the enone compound of formula (I) obtained by the method according to the invention.

In addition, in a third aspect, the invention relates to a washing or cleaning agent comprising the enone compound of formula (I) according to the invention.

Furthermore, in a fourth aspect, the invention relates to an insect repellent composition comprising the enone compound of formula (I) according to the invention.

In addition, in a fifth aspect, the invention pertains to a pharmaceutical composition comprising the enone compound of formula (I) according to the invention.

Finally, in a sixth aspect, the invention relates to a use of the enone compound of formula (I) according to the invention as fine chemical, basic chemical, insect repellant, pharmaceutical compound, fragrance or pro-fragrance compound.

"One or more", as used herein, relates to "at least one" and comprises 1, 2, 3, 4, 5, 6, 7, 8, 9 or more of the referenced species. Similarly, "at least one" means "one or more", i.e. 1, 2, 3, 4, 5, 6, 7, 8, 9 or more. "At least one", as used herein in relation to any component, refers to the number of chemically different atoms or molecules, i.e. to the number of different types of the referenced species, but not to the total number of atoms or molecules. For example, "at least one oxidant" means that at least one type of oxidant falling within the definition can be used in the method, but that also two or more different oxidant types falling within this definition can be present, but does not mean that several molecules of only one type of oxidant may be present.

Numeric values specified without decimal places refer to the full value specified with one decimal place. For example, "99 %" means "99.0 %", if not stated otherwise.

The expressions "approx." or "about", in conjunction with a numerical value, refer to a variance of ± 10 % relative to the given numerical value, preferably ± 5 %, more preferably ± 1 %, if not explicitly stated otherwise.

All mol% employed herein relate to the total amount of the respective starting materials, if not explicitly stated otherwise. The amount of the enone compound of formula (I) in washing or cleaning agents is indicated in weight percent (wt.-%), based on the total weight of the respective composition, if not explicitly stated otherwise.

These and other aspects, features and advantages of the invention become apparent to the skilled person in the following detailed description and claims. Each feature from one aspect of the invention can be used in any other aspect of the invention. Furthermore, the examples contained herein are intended to describe and illustrate the invention, but do not restrict it and in particular, the invention is not limited to these examples.

In particular, the invention pertains to:
1. A method for preparing an enone compound of formula (I) 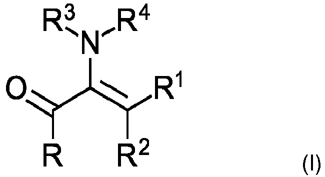 by reacting a ketone compound of formula (II) 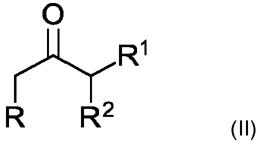 with an amine compound of formula (III) 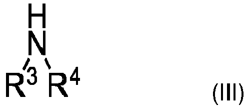 in the presence of at least one oxidant, preferably at a temperature in the range of 0 to 300 °C, more preferably in the range of 30 to 100 °C;
   wherein
   (1) R, R¹, R², R³ and R⁴ are independently of each other selected from hydrogen, substituted or unsubstituted linear C1-C20 alkyl groups or branched C3-C20 alkyl groups; substituted or unsubstituted linear C2-C20 alkenyl groups or branched C3-C20 alkenyl groups; substituted or unsubstituted linear C2-C20 alkynyl groups or branched C4-C20 alkynyl groups; substituted or unsubstituted C3-C10 cycloalkyl, C3-C10 cycloalkenyl or aryl groups; substituted or unsubstituted, linear C4-C20 alkylcycloalkyl, C4-C20 alkylcycloalkenyl, C4-C20 alkenylcycloalkyl, C4-C20 alkenylcycloalkenyl, C5-C20 alkynylcycloalkyl or C5-C20 alkynylcycloalkenyl groups, or branched C5-C20 alkylcycloalkyl, C5-C20 alkylcycloalkenyl, C5-C20 alkenylcycloalkyl, C5-C20 alkenylcycloalkenyl, C6-C20 alkynylcycloalkyl or C6-C20 alkynylcycloalkenyl groups; or substituted or unsubstituted linear C7-C20 alkylaryl, C8-C20 alkenylaryl or C8-C20 alkynylaryl groups, or branched C8-C20 alkylaryl, C8-C20 alkenylaryl or C9-C20 alkynylaryl groups; or substituted or unsubstituted linear C1-C20 alkylester, C1-C20 alkylether, or C1-C20 alkylmethoxy, or branched C2-C20 alkylester, C2-C20 alkylether, or C2-C20 alkylmethoxy groups; wherein each of the groups of R, R¹, R², R³ and R⁴ may further contain 1 to 6 heteroatoms, preferably 1, 2, 3, or 4 heteroatoms selected from O, N, S, and P, more preferably no heteroatom is present; or
      wherein
   (2) R and R² and/or R³ and R⁴ each form a substituted or unsubstituted hydrocarbon ring with 3 to 12 carbon atoms, preferably with 4 to 6 carbon atoms, more preferably with 4 or 5 carbon atoms,
      wherein each of the hydrocarbon rings may further contain at least one heteroatom, selected from O, N, S, and P, and/or wherein each of the hydrocarbon rings may further contain at least one double bond; and
      wherein the remaining groups are as defined above.
2. The method according to item 1, wherein R and R² form a substituted or unsubstituted hydrocarbon ring with 4 to 6 carbon atoms, preferably with 5 carbon atoms, wherein the hydrocarbon ring may contain at least one heteroatom selected from O, N, S, and P, and/or wherein the hydrocarbon ring may further contain at least one double bond; and
   wherein R¹ is preferably a substituted or unsubstituted linear C1-C15 alkyl, C2-C15 alkenyl, C2-C15 alkynyl, C4-C15 alkylcycloalkyl, C4-C15 alkylcycloalkenyl, C1-C15 alkylester, C1-C15 alkylether, or C1-C15 alkylmethoxy group, or branched C3-C15 alkyl, C3-C15 alkenyl, C4-C15 alkynyl, C5-C15 alkylcycloalkyl, C5-C15 alkylcycloalkenyl, C2-C15 alkylester, C2-C15 alkylether, or C2-C15 alkylmethoxy group, more preferably a substituted or unsubstituted linear C1-C8 alkyl, branched C3-C10 alkyl, linear C4-C15 alkylcycloalkenyl, branched C5-C15 alkylcycloalkenyl, linear C1-C15 alkylmethoxy, or branched C2-C15 alkylmethoxy group, wherein R¹ may further contain at least one heteroatom selected from O, N, S, and P; and wherein R³ and R⁴ are as defined in item 1.
3. The method according to item 1 and 2, wherein R³ and R⁴ form a substituted or unsubstituted pyrrolidine group;
   wherein preferably at least one hydrogen atom of the pyrrolidine ring is substituted with substituted or unsubstituted linear C1-C15 alkyl, C2-C15 alkenyl, C2-C15 alkynyl, C4-C15 alkylcycloalkyl, C4-C15 alkylcycloalkenyl, C1-C15 alkylester, C1-C15 alkylether, or C1-C15 alkylmethoxy groups, or branched C3-C15 alkyl, C3-C15 alkenyl, C4-C15 alkynyl, C5-C15 alkylcycloalkyl, C5-C15 alkylcycloalkenyl, C2-C15 alkylester, C2-C15 alkylether, or C2-C15 alkylmethoxy groups, more preferably with substituted or unsubstituted, linear C1-C8 alkyl, branched C3-C10 alkyl, linear C4-C15 alkylcycloalkenyl, branched C5-C15 alkylcycloalkenyl, linear C1-C15 alkylmethoxy, or branched C2-C15 alkylmethoxy groups, most preferably with -CH₂-O-CH₃,
   wherein the at least one substituent may further contain at least one heteroatom selected from O, N, S, and P.
4. The method according to any one of items 1 to 3, wherein the enone compound of formula (I) is an enone compound of formula (IV) 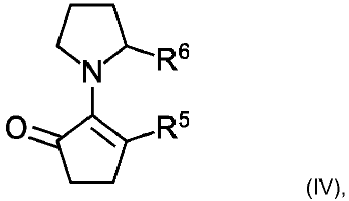 wherein
   (1) R⁵ and R⁶ are independently of each other selected from substituted or unsubstituted linear C1-C15 alkyl, C2-C15 alkenyl, C2-C15 alkynyl, C4-C15 alkylcycloalkyl, C4-C15 alkylcycloalkenyl, C1-C15 alkylester, C1-C15 alkylether, or C1-C15 alkylmethoxy groups, or branched C3-C15 alkyl, C3-C15 alkenyl, C4-C15 alkynyl, C5-C15 alkylcycloalkyl, C5-C15 alkylcycloalkenyl, C2-C15 alkylester, C2-C15 alkylether, or C2-C15 alkylmethoxy groups, preferably from substituted or unsubstituted, linear C1-C8 alkyl, branched C3-C10 alkyl, linear C4-C15 alkylcycloalkenyl, branched C5-C15 alkylcycloalkenyl, linear C1-C15 alkylmethoxy, or branched C2-C15 alkylmethoxy groups, wherein R⁵ and R⁶ may further contain independently of each other at least one heteroatom selected from O, N, S, and P; or wherein
   (2) R⁵ is a substituted or unsubstituted linear C1-C15 alkyl, branched C3-C15 alkyl, linear C4-C15 alkylcycloalkenyl, or branched C5-C15 alkylcycloalkenyl group; and
      wherein R⁶ is a substituted or unsubstituted linear C1-C15 alkylmethoxy, or branched C2-C15 alkylmethoxy group, preferably -CH₂-O-CH₃.
5. The method according to any one of items 1 to 4, wherein the enone compound of formula (I) is an enone compound of formula (V) or (VI) 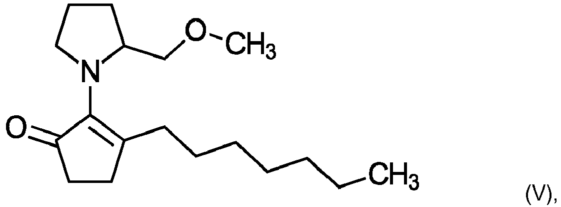 or 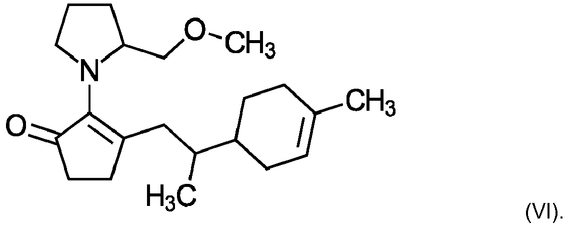
6. The method according to any one of items 1 to 5, wherein the reaction is conducted in the presence of at least one Bronsted acid or Lewis acid, preferably in the presence of para-toluene sulfonic acid.
7. The method according to any one of items 1 to 6, wherein the reaction is conducted in the presence of at least one catalyst, preferably in the presence of at least one main group metal based catalyst, transition metal based catalyst, organo-catalyst, or mixtures thereof, more preferably in the presence of at least one transition metal based catalyst, most preferably in the presence of at least one catalyst, which is based on Aluminum (Al), vanadium (V), chrome (Cr), manganese (Mn), iron (Fe), cobalt (Co), nickel (Ni), copper (Cu), zinc (Zn), zirconium (Zr), niobium (Nb), molybdenum (Mo), palladium (Pd), silver (Ag), tungsten (W), iridium (Ir), or platinum (Pt).
8. The method according to any one of items 1 to 7, wherein the oxidant is any compound, which is able to act as oxygen transfer reagent, preferably oxygen or air, more preferably air, most preferably air at atmospheric pressure.
9. The method according to any one of items 1 to 8, wherein the at least one catalyst is present in amounts of from 0.001 to 75 mol%, preferably in amounts of from 0.01 to 5 mol%, based on the total amount of starting materials.
10. The method according to any one of items 1 to 9, wherein the at least one Bronsted acid or Lewis acid is present in amounts of from 0.001 to 75 mol%, preferably in amounts of from 0.01 to 5 mol%, based on the total amount of starting materials.
11. An enone compound of formula (I) obtained by the method according to any one of items 1 to 10.
12. A washing or cleaning agent comprising the enone compound of formula (I) according to item 11.
13. An insect repellant composition comprising the enone compound of formula (I) according to item 11.
14. A pharmaceutical composition comprising the enone compound of formula (I) according to item 11.
15. Use of the enone compound of formula (I) according to item 11 as fine chemical, basic chemical, insect repellant, pharmaceutical compound, fragrance or pro-fragrance compound.

According to the invention, when one or more groups of R, R¹, R², R³, R⁴, R⁵ and R⁶ are substituted, the substituent can be chosen from every suitable substituent, which is known to the skilled person. Preferably, each of the groups of R, R¹, R², R³, R⁴, R⁵ and R⁶ may independently of each other contain 1 to 10 of one or more of the following substituents: halogen, ester, ether, hydroxyl, amine, amide, ketone, aldehyde, carboxyl, linear C1-C20 alkyl, branched C3-C20 alkyl, linear C2-C20 alkenyl, branched C3-C20 alkenyl, linear C2-C20 alkynyl, branched C4-C20 alkynyl; C3-C20 cycloalkyl, C3-C20 cycloalkenyl, or aryl; linear C4-C20 alkylcycloalkyl(alkyl), C4-C20 alkylcycloalkenyl(alkyl), C4-C20 alkenylcycloalkyl(alkyl), C4-C20 alkenylcycloalkenyl(alkyl), C5-C20 alkynylcycloalkyl(alkyl) or C5-C20 alkynylcycloalkenyl(alkyl), or branched C5-C20 alkylcycloalkyl(alkyl), C5-C20 alkylcycloalkenyl(alkyl), C5-C20 alkenylcycloalkyl(alkyl), C5-C20 alkenylcycloalkenyl(alkyl), C6-C20 alkynylcycloalkyl(alkyl) or C6-C20 alkynylcycloalkenyl(alkyl); or linear C7-C20 alkylaryl(alkyl), C8-C20 alkenylaryl(alkyl) or C8-C20 alkynylaryl(alkyl), or branched C8-C20 alkylaryl(alkyl), C8-C20 alkenylaryl(alkyl) or C9-C20 alkynylaryl(alkyl), or linear C1-C15 alkylester(alkyl), C1-C15 alkylether(alkyl), or C1-C15 alkylmethoxy groups, or branched C2-C15 alkylester(alkyl), C2-C15 alkylether(alkyl), or C2-C15 alkylmethoxy groups, or combinations thereof. The substituents may contain one or more heteroatom(s), such as, O, N, S, and P. The substituents may replace one or more of -H, -C-, -CH-, -CH₂- and/or -CH₃ in the groups of R, R¹, R², R³, R⁴, R⁵ and R⁶ or in the formed rings of R and R² and/or R³ and R⁴. Preferred substituents are linear C1-C10 alkyl, preferably methyl and ethyl, methylmethoxy (-CH₂-O-CH₃), and linear C4-C20 alkylcycloalkenyl(alkyl) or branched C5-C20 alkylcycloalkenyl(alkyl) groups. Most preferably, the pyrrolidine ring is substituted by a methylmethoxy group (-CH₂-O-CH₃).

In various embodiments, the substituent cannot be the same group as the group being substituted. For example, this means when the substituted group is an alkyl group, the substituent is not itself an alkyl group. This similarly applies to all other combinations of substituted group and substituent.

The minimum and maximum limit of carbon atoms (CX-CX) of a group of R, R¹, R², R³, R⁴, R⁵ and R⁶ relates to the total amount of carbon atoms of a group of R, R¹, R², R³, R⁴, R⁵ and R⁶ without any optional substituent. That means that optional present substituents of a group of R, R¹, R², R³, R⁴, R⁵ and R⁶ may increase the total number of carbon atoms over the indicated minimum and maximum limit of carbon atoms of the respective group. In various other embodiments, the given number of carbon atoms also includes all potential substituents.

The method for preparing an enone compound of formula (I) according to the invention is exemplarily shown in the following reaction scheme:

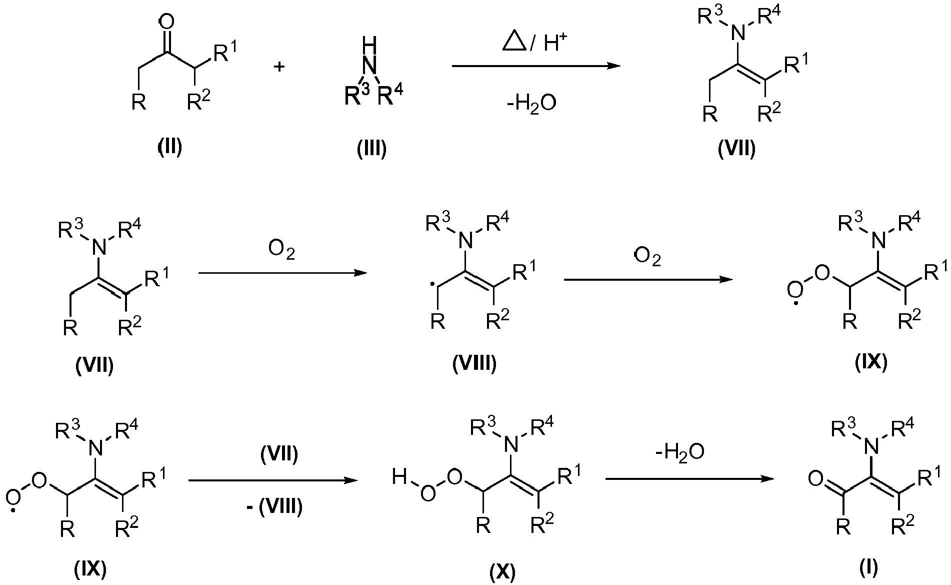

Starting from a ketone compound of formula (II) and an amine compound of formula (III) in the presence of at least one oxidant, the enone compound of formula (I) is formed. Not to be bound to any theory, it is believed that this reaction takes place, since the ketone compound of formula (II) contains a CH₂ group in alpha position to the ketone group.

The reaction is conducted in the presence of an oxidant, which is preferably any compound, which is able to act as oxygen transfer reagent, more preferably oxygen or air, more preferably air, most preferably air at atmospheric pressure.

In another preferred embodiment, the reaction is further conducted at temperatures in the range of 0 to 300 °C, more preferably in the range of 30 to 100 °C, most preferably in the range of 50 to 80 °C.

In a further preferred embodiment, the reaction of the method according to the invention is conducted in the presence of at least one Brønsted acid or Lewis acid.

Lewis acids are electrophile electron pair acceptors, for example compounds with incomplete or unstable electron octets, such as B(CH₃)₃, BF₃, or AlCl₃, or compounds which are not present in nobel gas configuration. Furthermore, Lewis acids can be metal cations, which can be used as central atom in complex compounds, or molecules with polarized double bonds, such as CO₂ or SO₃, or unsaturated halides, such as SiCl₄.

Brønsted acids are proton donors. These acids release protons to a corresponding base. Preferred Brønsted acids are CH₃COOH, H₂SO₄, HSO₄, toluene sulfonic acid, HPO₄²⁻, H₂PO₄⁻, HCl, H₂O, NH₃ and NH₄⁺.

A preferred Brønsted acid is para-toluene sulfonic acid.

In a further preferred embodiment, the at least one Brønsted acid or Lewis acid is present in amounts of from 0.001 to 75 mol%, more preferably in amounts of from 0.01 to 5 mol%, based on the total amount of starting materials.

In a further preferred embodiment, the reaction is conducted in the presence of at least one catalyst, preferably in the presence of at least one main group metal based catalyst, transition metal based catalyst, organo catalyst, or mixtures thereof.

Metals, which can be used for main group metal based catalysts are preferably from Groups 1, 2, 13, and 14 (periodic table of elements) and may be in its highest state of oxidation. In preferred embodiments, the metal atom is selected from the group comprising lithium (Li), beryllium (Be), sodium (Na), magnesium (Mg), potassium (K), calcium (Ca), boron (B), aluminum (Al), gallium (Ga), indium (In), silicon (Si), germanium (Ge), and tin (Sn). In more preferred embodiments, the metal is boron and aluminum.

Suitable transition metal based catalyst, are based on transition metals from the group of vanadium (V), chrome (Cr), manganese (Mn), iron (Fe), cobalt (Co), nickel (Ni), copper (Cu), zinc (Zn), zirconium (Zr), niobium (Nb), molybdenum (Mo), palladium (Pd), silver (Ag), tungsten (W), iridium (Ir), or platinum (Pt).

Suitable organo catalysts are catalysts, which do not contain metal atoms. Preferably, these catalysts are based on phosphor (P), sulfur (S), nitrogen (N), oxygen (O), carbon (C) and hydrogen (H).

In preferred embodiments, the at least one catalyst used in the reaction is a transition metal based catalyst.

Suitable catalysts are for example solid supported Cu-catalysts as disclosed in RSC Advances, 5(106), 87221-87227; 2015, silica supported strong Lewis acid AlCl₃ for example disclosed in RSC Advances, 6(14), 11528-11535; 2016); Pd₂(dba)₃ via a triflate for example disclosed in Macromolecular Chemistry and Physics, 211(21), 2339-2346; 2010, Co(OAc)₂ on 1,4 benzochinone for example disclosed in Angewandte Chemie, International Edition, 57(31), 9805-9809; 2018.

Further preferred, the at least one catalyst is present in amounts of from 0.001 to 75 mol%, more preferably in amounts of from 0.01 to 5 mol%, based on the total amount of starting materials.

According to the second aspect of the invention, the enone compound of formula (I) 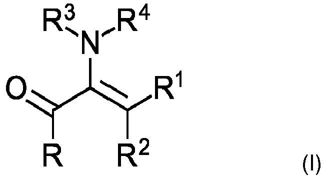 obtained or obtainable by the method according to the invention is claimed, wherein
(1) R, R¹, R², R³ and R⁴ are independently of each other selected from hydrogen, substituted or unsubstituted linear C1-C20 alkyl groups or branched C3-C20 alkyl groups; substituted or unsubstituted linear C2-C20 alkenyl groups or branched C3-C20 alkenyl groups; substituted or unsubstituted linear C2-C20 alkynyl groups or branched C4-C20 alkynyl groups; substituted or unsubstituted C3-C10 cycloalkyl, C3-C10 cycloalkenyl or aryl groups; substituted or unsubstituted, linear C4-C20 alkylcycloalkyl, C4-C20 alkylcycloalkenyl, C4-C20 alkenylcycloalkyl, C4-C20 alkenylcycloalkenyl, C5-C20 alkynylcycloalkyl or C5-C20 alkynylcycloalkenyl groups, or branched C5-C20 alkylcycloalkyl, C5-C20 alkylcycloalkenyl, C5-C20 alkenylcycloalkyl, C5-C20 alkenylcycloalkenyl, C6-C20 alkynylcycloalkyl or C6-C20 alkynylcycloalkenyl groups; or substituted or unsubstituted linear C7-C20 alkylaryl, C8-C20 alkenylaryl or C8-C20 alkynylaryl groups, or branched C8-C20 alkylaryl, C8-C20 alkenylaryl or C9-C20 alkynylaryl groups; or substituted or unsubstituted linear C1-C20 alkylester, C1-C20 alkylether, or C1-C20 alkylmethoxy, or branched C2-C20 alkylester, C2-C20 alkylether, or C2-C20 alkylmethoxy groups; wherein each of the groups of R, R¹, R², R³ and R⁴ may further contain 1 to 6 heteroatoms, preferably 1, 2, 3, or 4 heteroatoms selected from O, N, S, and P; or
   wherein
(2) R and R² and/or R³ and R⁴ each form a substituted or unsubstituted hydrocarbon ring with 3 to 12 carbon atoms, preferably with 4 to 6 carbon atoms, more preferably with 4 or 5 carbon atoms,
   wherein each of the hydrocarbon rings may further contain at least one heteroatom, selected from O, N, S, and P, and/or wherein each of the hydrocarbon rings may further contain at least one double bond; and
   wherein the remaining groups are as defined above.

In a preferred embodiment, R and R² of the enone compound of formula (I) form a substituted or unsubstituted hydrocarbon ring with 4 to 6 carbon atoms, preferably with 5 carbon atoms, wherein the hydrocarbon ring may contain at least one heteroatom selected from O, N, S, and P, and/or wherein the hydrocarbon ring may further contain at least one double bond; and
wherein R¹ is preferably a substituted or unsubstituted linear C1-C15 alkyl, C2-C15 alkenyl, C2-C15 alkynyl, C4-C15 alkylcycloalkyl, C4-C15 alkylcycloalkenyl, C1-C15 alkylester, C1-C15 alkylether, or C1-C15 alkylmethoxy group, or branched C3-C15 alkyl, C3-C15 alkenyl, C4-C15 alkynyl, C5-C15 alkylcycloalkyl, C5-C15 alkylcycloalkenyl, C2-C15 alkylester, C2-C15 alkylether, or C2-C15 alkylmethoxy group, more preferably a substituted or unsubstituted linear C1-C8 alkyl, branched C3-C10 alkyl, linear C4-C15 alkylcycloalkenyl, branched C5-C15 alkylcycloalkenyl, linear C1-C15 alkylmethoxy, or branched C2-C15 alkylmethoxy group, wherein R¹ may further contain at least one heteroatom selected from O, N, S, and P; and wherein R³ and R⁴ are as defined in aspect 2.

In a preferred embodiment, R¹ is substituted or unsubstituted linear C1-C10 alkyl or branched C3-C10 alkyl, substituted or unsubstituted linear C4-C15 alkylcycloalkenyl or branched C5-C15 alkylcycloalkenyl. Most preferably, R¹ is unsubstituted linear C1-C10 alkyl or branched C5-C15 alkylcycloalkenyl(alkyl), in particular 2-(4-methylcyclohex-3-enyl)propyl.

In another preferred embodiment, R³ and R⁴ of the enone compound of formula (I) form a substituted or unsubstituted pyrrolidine group;
wherein preferably at least one hydrogen atom of the pyrrolidine ring is substituted with substituted or unsubstituted linear C1-C15 alkyl, C2-C15 alkenyl, C2-C15 alkynyl, C4-C15 alkylcycloalkyl, C4-C15 alkylcycloalkenyl, C1-C15 alkylester, C1-C15 alkylether, or C1-C15 alkylmethoxy groups, or branched C3-C15 alkyl, C3-C15 alkenyl, C4-C15 alkynyl, C5-C15 alkylcycloalkyl, C5-C15 alkylcycloalkenyl, C2-C15 alkylester, C2-C15 alkylether, or C2-C15 alkylmethoxy groups, more preferably with substituted or unsubstituted, linear C1-C8 alkyl, branched C3-C10 alkyl, linear C4-C15 alkylcycloalkenyl, branched C5-C15 alkylcycloalkenyl, linear C1-C15 alkylmethoxy, or branched C2-C15 alkylmethoxy groups, most preferably with -CH₂-O-CH₃,
wherein the at least one substituent may further contain at least one heteroatom selected from O, N, S, and P.

In a further preferred embodiment, the enone compound of formula (I) according to the invention is an enone compound of formula (IV) 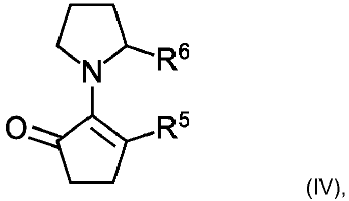 wherein
(1) R⁵ and R⁶ are independently of each other selected from substituted or unsubstituted linear C1-C15 alkyl, C2-C15 alkenyl, C2-C15 alkynyl, C4-C15 alkylcycloalkyl, C4-C15 alkylcycloalkenyl, C1-C15 alkylester, C1-C15 alkylether, or C1-C15 alkylmethoxy groups, or branched C3-C15 alkyl, C3-C15 alkenyl, C4-C15 alkynyl, C5-C15 alkylcycloalkyl, C5-C15 alkylcycloalkenyl, C2-C15 alkylester, C2-C15 alkylether, or C2-C15 alkylmethoxy groups, preferably from substituted or unsubstituted, linear C1-C8 alkyl, branched C3-C10 alkyl, linear C4-C15 alkylcycloalkenyl, branched C5-C15 alkylcycloalkenyl, linear C1-C15 alkylmethoxy, or branched C2-C15 alkylmethoxy groups, wherein R⁵ and R⁶ may further contain independently of each other at least one heteroatom selected from O, N, S, and P; or
   wherein
(2) R⁵ is a substituted or unsubstituted linear C1-C15 alkyl, branched C3-C15 alkyl, linear C4-C15 alkylcycloalkenyl, or branched C5-C15 alkylcycloalkenyl group; and
   wherein R⁶ is a substituted or unsubstituted linear C1-C15 alkylmethoxy, or branched C2-C15 alkylmethoxy group, preferably -CH₂-O-CH₃.

In a more preferred embodiment, the enone compound of formula (I) according to the invention is an enone compound of formula (V) or (VI) 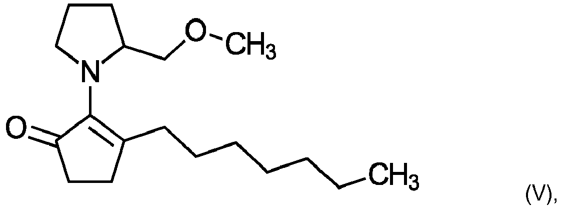 or

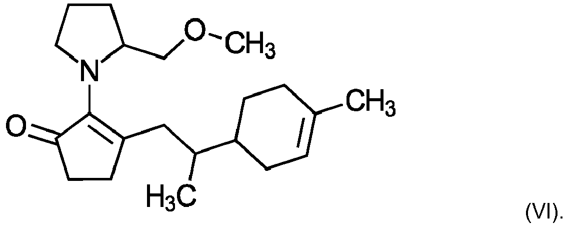

According to aspect 3 of the invention, a washing or cleaning agent comprising the enone compound of formula (I) obtained by the method according to the invention is claimed.

The washing or cleaning agent can be a solid or liquid composition at 20 °C and atmospheric pressure.

Preferably, washing or cleaning agents comprise laundry detergents, laundry after treatment agents, home care products, such as dishwashing detergent, floor cleaning agent or window cleaner, air care products, or cosmetic products.

The washing or cleaning agents may contain at least one further component, preferably selected from the group of anionic surfactants, cationic surfactants, amphoteric surfactants, nonionic surfactants, acidifiers, alkalizing agents, anti-crease compounds, antibacterial/antimicrobial substances, antioxidants, anti-deposition agents, antistatic agents, bitter substances, bleaching agents, bleach activators, bleach stabilizers, bleach catalysts, builder substances, corrosion inhibitors, ironing aids, co-builders, further fragrances or pro-fragrances, shrinkage inhibitors, electrolytes, emulsifiers, enzymes, enzyme stabilizers, protease inhibitors, colorants, dyes, dye transfer inhibitors, fluorescent agents, fungicides, germicides, metal sequestering agents, odor-complexing substances, auxiliary agents, hydrotropics, rinse aids, complexing agents, preservatives, optical brighteners, perfume carriers, pearl-luster agents, pH control agents, phobing and impregnating agents, polymers, swelling and sliding fasteners, foam inhibitors, layered silicates, dirt-repellent substances, organic solvents, silicone oils, soil-release active substances, UV-protective substances, viscosity regulators, thickeners, discoloration inhibitors, greying inhibitors, vitamins, fabric softeners and water, with the proviso that the at least one further component is different from the enone compound of formula (I) according to the invention.

Referring to aspect 6 of the invention, the enone compound of formula (I) obtained by the method according to the invention, can be used as fine chemical, basic chemical, pharmaceutical compound, fragrance or pro-fragrance compound.

In preferred embodiments, the enone compound of formula (I) is a fragrance or pro-fragrance compound, in particular when used in washing or cleaning agents according to the invention.

Pro-fragrance compounds are able to release a fragrance compound, which provides a pleasant scent. In some embodiments, the fragrance compound is a ketone or diketone compound derived from formula (II) according to the invention. Suitable ketones are known to those skilled in the art and include those satisfying the struictural requirements of formula (II) and being described in US 2003/0158079 A1, paragraphs [0154] and [0155] as well as in Steffen Arctander, Aroma Chemicals Vol. 1 and Vol. 2 (published 1960 and 1969, new edition 2000; ISBN: 0-931710-37-5 und 0-931710-38-3).

Further suitable fragrance ketones include those that satisfy the structural criteria of formula (II) and are selected from, but not limited to, the following: methyl beta naphthyl ketone, musk indanon (1,2,3,5,6,7-hexahydro-1,1,2,3,3-pentamethyl-4H-indene-4-on), calone (methylbenzodioxepinone), tonalid (6-acetyl-1,1,2,4,4,7-hexamethyltetraline), alpha-damascone, beta-damascone, delta-damascone, iso-damascone, damascenone, methyl dihydrojasmonate (Hedion), menthone, carvon, camphor, coavon (3,4,5,6,6-pentamethylhept-3-en-2-on), fenchone, alpha-ionone, beta-ionone, dihydro-beta-ionone, gamma-methyl-ionone, fleuramone (2-heptylcyclopentanone), frambinon methyl ether (4-(4-methoxyphenyl)butane-2-one), dihydrojasmone, cis-jasmone, 1-(1,2,3,4,5,6,7,8-octahydro-2,3,8,8-tetramethyl-2-naphthalenyl)-ethane-1-one and isomers thereof, methylcedrenylketone, acetophenone, methylacetophenone, para-methoxyacetophenone, methyl-beta-naphtylketone, benzeneacetone, benzophenone, para-hydroxyphenylbutanone, 3-methyl-5-propyl-2-cyclohexenone, 6-isopropyldeca-hydro-2-naphtone, dimethyloctenone, frescomenthe (2-butane-2-ylcyclohexane-1-one), 4-(1-ethoxyvinyl)-3,3,5,5-tetramethylcyclohexanone, methylheptenone, 2-(2-(4-methyl-3-cyclohexene-1-yl)propyl)cyclopentanone, 1-(p-menthen-6(2)yl)-1-propanone, 4-(4-hydroxy-3-methoxyphenyl)-2-butanone, 2-acetyl-3,3-dimethylnorbornane, 6,7-dihydro-1,1,2,3,3-pentamethyl-4(5H)indanone, 4-damascol, dulcinyl (4-(1,3-Benzodioxol-5-yl)butane-2-one), hexalone (1-(2,6,6-trimethyl-2-cyclohexene-1-yl)-1,6-heptadiene-3-one), isocyclemon E (2-acetonaphthone-1,2,3,4,5,6,7,8-octahydro-2,3,8,8-tetramethyl), methylnonylketone, methylcyclocitrone, methyllavenderketone, orivon (4-tert-amylcyclohexanone), 4-tert-butylcyclohexanone, delphon (2-pentylcyclopentanone), muscon (CAS 541 -91 -3), neobutenone (1-(5,5-dimethyl-1-cyclo-hexenyl)pent-4-ene-1-one), Plicaton (CAS 41724-19-0), Velouton (2,2,5-trimethyl-5-pentylcyclopentane-1-one), 2,4,4,7-tetramethyl-oct-6-ene-3-one, tetrameran (6,10-dimethylundecene-2-one) and mixtures thereof.

In a preferred embodiment, the fragrance compound is released from the pro-fragrance compound of formula (I) by contact with water, oxygen, sunlight, elevated temperatures or by contact with the skin.

Suitable amounts of the enone compound of formula (I) and of further components for the compositions are based on the application of the washing or cleaning agent according to the invention and are known to the skilled person.

If the washing agent according to the invention is a laundry detergent, the enone compound of formula (I) may be present in the washing agent in amounts of from 0.001 to 5 wt.-%, more preferably of from 0.01 to 2 wt.-%, based on the total weight of the composition.

In case the washing or cleaning agent is an air care product, the enone compound of formula (I) may be present in the washing agent in amounts of from 0.001 to 99.9 wt.-%, more preferably of from 0.01 to 50 wt.-%, based on the total weight of the composition.

According to a fourth aspect, the invention pertains to an insect repellant composition comprising the enone compound of formula (I) obtained by the method according to the invention.

In one preferred embodiment, the amount of enone compound of formula (I) according to the present invention is in the range of 0.001 to 99.9 wt.-%, more preferably in the range of 0.01 to 85 wt.-%, more preferably in the range of 0.1 to 70 wt.-%, most preferably in the range of 1 to 50 wt.-%, based on the total weight of the insect repellant composition.

In various embodiments the insect repellent composition can comprise further additives, which are common in the field of insect repellant. For examples the additives which are disclosed for washing or cleaning agents can be employed as well.

In a fifth aspect, the invention pertains to a pharmaceutical composition comprising the enone compound of formula (I) obtained by the method according to the invention.

In one preferred embodiment, the amount of enone compound of formula (I) according to the present invention is in the range of 0.001 to 99.9 wt.-%, more preferably in the range of 0.01 to 85 wt.-%, more preferably in the range of 0.1 to 50 wt.-%, most preferably in the range of 1 to 15 wt.-%, based on the total weight of the pharmaceutical composition.

In various embodiments the pharmaceutical composition can comprise further additives, which are common in the field of pharmaceutical compositions.

In a preferred embodiment, enone compounds of formula (I) are used as fine or basic chemicals. Preferably, they are contained in the chemical composition in amounts of at least 90 weight-%, preferably of at least 95 wt.-%, more preferably of at least 99 wt.-%, more preferably of at least 99.5 wt.-%, most preferably of at least 99.9 wt.-%, based on the total weight of the fine or basic chemical.

The chemical composition may be a solid or a liquid composition at 20 °C and atmospheric pressure.

The invention will now be described in greater detail, with reference to the accompanying non-limiting examples. It is understood that all embodiments disclosed herein in relation to the method according to the invention are similarly applicable to the enone compound of formula (I), to the washing or cleaning agents according to the invention and to the use of the enone compound of formula (I), and vice versa. Specific embodiments and features of the embodiments are to be seen as disclosed for each and every embodiment so that features disclosed in the specification can be combined with each other with the embodiment still being within the scope of the present invention.

In the following examples, the present invention is further described in preferred embodiments but it is understood that it is not limited thereto.

### Examples

### General procedure:

The respective ketone (1 eq.), the respective pyrrolidine (1.9 eq - 2.5 eq.), and para-toluene sulfonic acid (0.1 mol%) in toluene (150 mL) were refluxed with azeotropic removal of H₂O in a Dean-Stark trap over time. The reaction mixture was then cooled to room temperature, washed with water and dried over Na₂SO₄, filtered and the solvent was evaporated under reduced pressure. The pure products were isolated via column chromatography (hexane - ethyl acetate - 1 % triethylamine) unless otherwise noted.

### Example 1: 2-[2-(methoxymethyl)pyrrolidin-1-yl]-3-heptylcyclopent-2-en-1-one

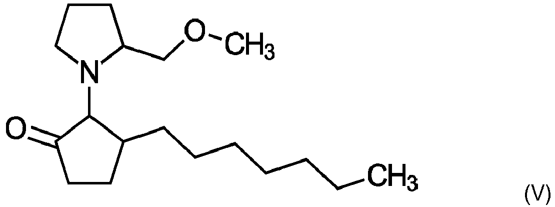

**2-[2-(methoxymethyl)pyrrolidin-1-yl]-3-heptylcyclopent-2-en-1-one** was prepared following the aforementioned method using 2-heptylcyclopentanone (3.27 g, 18 mmol) and 2-methoxymethylpyrrolidine (4.05 g, 35 mmol). Until full conversion of the starting material (approx. 120 hours), the reaction was monitored by gas chromatography (GC).
Pale yellow oil, 11 % yield. R_{f} = 0.19 (10 % ethyl acetate in hexane +1 % Et₃N). FTIR (film): *ṽ* = 2924, 2855, 1696, 1635, 1612, 1458, 1376, 1300, 1254, 1221, 1198, 1109, 1014, 972, 916, 819, 724, 675, 573 cm⁻¹. ¹H NMR (400 MHz, CDCl₃): δ = 0.89 (t, *J* = 7.0 Hz, 3H), 1.22-1.38 (m, 8H), 1.45-1.55 (m, 2H), 1.64-1.72 (m, 1H), 1.80-1.89 (pseudo-quint, *J* = 6.6 Hz, 7.0 Hz, 2H), 1.99-2.09 (m, 1H), 2.30-2.34 (bd*, *J* = 4.9 Hz, 2H), 2.41-2.48 (m, 4H), 3.02 (q*, *J* = 7.1 Hz, 1H), 3.10 (dd, *J* = 6.9 Hz, 9.3 Hz, 1H), 3.18 (dd, *J* = 4.7 Hz, 9.3 Hz, 1H), 3.26 (q, *J* = 6.3 Hz, 1H), 3.28 (s, 3H), 4.04-4.11 (m, 1H).
¹³C-NMR (CDCl₃, 125.8 MHz): δ = 14.1 (q), 22.6 (t), 24.5 (t), 27.0 (t), 27.7 (t), 28.9 (t), 29.1 (t), 29.7 (t), 30.7 (t), 31.8 (t), 34.0 (t), 51.9 (t), 57.8 (d), 58.9 (q), 76.3 (t), 144.2 (s), 165.0 (s), 207.5 (s). GC-MS: Optima 5 Accent 815, He, Split 1:10; 50°C-12°C/min-320°C; *t_{R}* = 16.1 min. MS (EI, 70 eV) *m*/*z* (%) at 16.1 min: 293 (1) [M]⁺, 248 (100) [M-C₂H₅O]⁺, 190 (4), 176 (5), 134 (5), 120 (4), 41 (5).
* shows fine structure.

### Example 2: 2-[2-(methoxymethyl)pyrrolidin-1-yl]-3-{2-[2-(4-methylcyclohex-3-en-1-yl)propyl]}cyclo pent-2-en-1-one

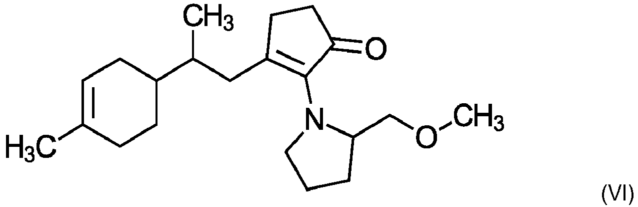

**2-[2-(methoxymethyl)pyrrolidin-1-yl]-3-{2-[2-(4-methylcyclohex-3-en-1-yl)propyl]}cyclopent-2-en-1-one** was prepared following the aforementioned method using 2-[2-(4-methylcyclohex-3-en-1-yl)propyl]cyclopentanone (6.4 g, 29 mmol) and 2-methoxymethylpyrrolidine (8.0 g, 69 mmol). Until full conversion of the starting material (approx. 175 hours), the reaction was monitored by GC.
Pale yellow oil, 11 % yield. R_{f} = 0.19 (10 % EtOAc in hexane +1% Et₃N). FTIR (film) 2 diastereoisomers: *ṽ* = 2959, 2916, 2872, 2834, 1696, 1633, 1448, 1377, 1198, 1109, 972, 913, 798, 673, 575, 427 cm⁻¹.
¹H NMR (400 MHz, CDCl₃) 2 diastereoisomers: δ = 0.82 (d, *J* = 6.8 Hz, 10.2 Hz, isomer A, 3H)/ 0.84 (d, *J* = 6.8 Hz, 10.2 Hz, isomer B, 3H), 1.18-1.48 (m, 3H), 1.62 (s, 3H), 1.60-1.70 (m, 2H), 1.70-1.88 (m, 2H), 1.84 (t, *J* = 7.1 Hz, 3H), 1.88-2.10 (m, 4H), 2.28-2.52 (m, 6H), 3.02 (q, J = 7.1 Hz, 1H), 3.04-3.24 (m, 3H), 3.24 (s, 3H), 4.01-4.09 (m, 1H), 5.36 (bs, 1H).
¹³C-NMR (CDCl₃, 125.8 MHz) 2 diastereoisomers: δ = 16.0 (q)/ 16.2 (q), 23.4 (2 isomers, q), 24.5 (t)/ 24.6 (t), 24.9 (t), 27.0 (t)/ 27.2 (t), 27.4/ 27.5 (t), 29.0 (2 isomers, t), 29.7 (t), 30.7 (t)/ 30.8 (t), 34.0 (2 isomers, t), 35.0 (t)/ 35.3 (t), 35.6 (d)/ 35.8 (d), 38.3 (d)/ 38.5 (d), 52.0 (2 isomers, t), 57.8 (2 isomers, d), 58.9 (q)/ 58.9 (q), 76.4 (t)/ 76.5 (t), 120.7 (2 isomers, d), 133.9 (s)/ 134.1 (s), 145.4 (s)/ 145.5 (s), 165.3 (s)/ 165.7 (s), 207.5 (2 isomers, s). ESI (positive ion) *m*/*z* (%): 408 (40), 354 (16), 332 (100) [M+H]+, 300 (53). GC-MS: Optima 5 Accent 815, He, Split 1:10; 50°C-12°C/min-320°C; *t_{R}* = 18.69 min, 18.76 min. MS (EI, 70 eV) *m*/*z* (%) for both isomers similar: 331 (6) [M]⁺, 286 (100) [M-C₂H₅O]⁺, 204 (9), 164 (26), 121 (19), 95 (9) [M-C₁₄H₂₂NO₂]⁺, 67 (13), 41 (10).

## Claims

1. A method for preparing an enone compound of formula (I) 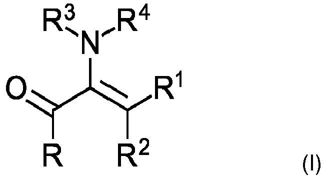 by reacting a ketone compound of formula (II) 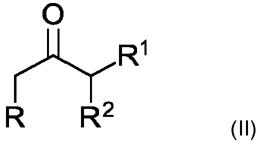 with an amine compound of formula (III) 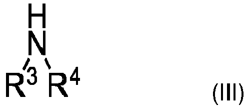 in the presence of at least one oxidant, preferably at a temperature in the range of 0 to 300 °C, more preferably in the range of 30 to 100 °C;
wherein
(1) R, R¹, R², R³ and R⁴ are independently of each other selected from hydrogen, substituted or unsubstituted linear C1-C20 alkyl groups or branched C3-C20 alkyl groups; substituted or unsubstituted linear C2-C20 alkenyl groups or branched C3-C20 alkenyl groups; substituted or unsubstituted linear C2-C20 alkynyl groups or branched C4-C20 alkynyl groups; substituted or unsubstituted C3-C10 cycloalkyl, C3-C10 cycloalkenyl or aryl groups; substituted or unsubstituted, linear C4-C20 alkylcycloalkyl, C4-C20 alkylcycloalkenyl, C4-C20 alkenylcycloalkyl, C4-C20 alkenylcycloalkenyl, C5-C20 alkynylcycloalkyl or C5-C20 alkynylcycloalkenyl groups, or branched C5-C20 alkylcycloalkyl, C5-C20 alkylcycloalkenyl, C5-C20 alkenylcycloalkyl, C5-C20 alkenylcycloalkenyl, C6-C20 alkynylcycloalkyl or C6-C20 alkynylcycloalkenyl groups; or substituted or unsubstituted linear C7-C20 alkylaryl, C8-C20 alkenylaryl or C8-C20 alkynylaryl groups, or branched C8-C20 alkylaryl, C8-C20 alkenylaryl or C9-C20 alkynylaryl groups; or substituted or unsubstituted linear C1-C20 alkylester, C1-C20 alkylether, or C1-C20 alkylmethoxy, or branched C2-C20 alkylester, C2-C20 alkylether, or C2-C20 alkylmethoxy groups; wherein each of the groups of R, R¹, R², R³ and R⁴ may further contain 1 to 6 heteroatoms, preferably 1, 2, 3, or 4 heteroatoms selected from O, N, S, and P; or
wherein
(2) R and R² and/or R³ and R⁴ each form a substituted or unsubstituted hydrocarbon ring with 3 to 12 carbon atoms, preferably with 4 to 6 carbon atoms, more preferably with 4 or 5 carbon atoms,
wherein each of the hydrocarbon rings may further contain at least one heteroatom, selected from O, N, S, and P, and/or wherein each of the hydrocarbon rings may further contain at least one double bond; and
wherein the remaining groups are as defined above.

2. The method according to claim 1, wherein R and R² form a substituted or unsubstituted hydrocarbon ring with 4 to 6 carbon atoms, preferably with 5 carbon atoms, wherein the hydrocarbon ring may contain at least one heteroatom selected from O, N, S, and P, and/or wherein the hydrocarbon ring may further contain at least one double bond; and
wherein R¹ is preferably a substituted or unsubstituted linear C1-C15 alkyl, C2-C15 alkenyl, C2-C15 alkynyl, C4-C15 alkylcycloalkyl, C4-C15 alkylcycloalkenyl, C1-C15 alkylester, C1-C15 alkylether, or C1-C15 alkylmethoxy group, or branched C3-C15 alkyl, C3-C15 alkenyl, C4-C15 alkynyl, C5-C15 alkylcycloalkyl, C5-C15 alkylcycloalkenyl, C2-C15 alkylester, C2-C15 alkylether, or C2-C15 alkylmethoxy group, more preferably a substituted or unsubstituted linear C1-C8 alkyl, branched C3-C10 alkyl, linear C4-C15 alkylcycloalkenyl, branched C5-C15 alkylcycloalkenyl, linear C1-C15 alkylmethoxy, or branched C2-C15 alkylmethoxy group, wherein R¹ may further contain at least one heteroatom selected from O, N, S, and P; and wherein R³ and R⁴ are as defined in claim 1.

3. The method according to claim 1 and 2, wherein R³ and R⁴ form a substituted or unsubstituted pyrrolidine group;
wherein preferably at least one hydrogen atom of the pyrrolidine ring is substituted with substituted or unsubstituted linear C1-C15 alkyl, C2-C15 alkenyl, C2-C15 alkynyl, C4-C15 alkylcycloalkyl, C4-C15 alkylcycloalkenyl, C1-C15 alkylester, C1-C15 alkylether, or C1-C15 alkylmethoxy groups, or branched C3-C15 alkyl, C3-C15 alkenyl, C4-C15 alkynyl, C5-C15 alkylcycloalkyl, C5-C15 alkylcycloalkenyl, C2-C15 alkylester, C2-C15 alkylether, or C2-C15 alkylmethoxy groups, more preferably with substituted or unsubstituted, linear C1-C8 alkyl, branched C3-C10 alkyl, linear C4-C15 alkylcycloalkenyl, branched C5-C15 alkylcycloalkenyl, linear C1-C15 alkylmethoxy, or branched C2-C15 alkylmethoxy groups, most preferably with -CH₂-O-CH₃,
wherein the at least one substituent may further contain at least one heteroatom selected from O, N, S, and P.

4. The method according to any one of claims 1 to 3, wherein the enone compound of formula (I) is an enone compound of formula (IV) 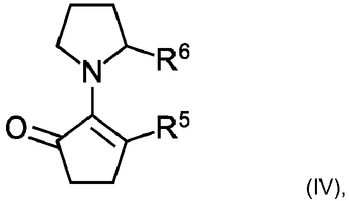 wherein
(1) R⁵ and R⁶ are independently of each other selected from substituted or unsubstituted linear C1-C15 alkyl, C2-C15 alkenyl, C2-C15 alkynyl, C4-C15 alkylcycloalkyl, C4-C15 alkylcycloalkenyl, C1-C15 alkylester, C1-C15 alkylether, or C1-C15 alkylmethoxy groups, or branched C3-C15 alkyl, C3-C15 alkenyl, C4-C15 alkynyl, C5-C15 alkylcycloalkyl, C5-C15 alkylcycloalkenyl, C2-C15 alkylester, C2-C15 alkylether, or C2-C15 alkylmethoxy groups, preferably from substituted or unsubstituted, linear C1-C8 alkyl, branched C3-C10 alkyl, linear C4-C15 alkylcycloalkenyl, branched C5-C15 alkylcycloalkenyl, linear C1-C15 alkylmethoxy, or branched C2-C15 alkylmethoxy groups, wherein R⁵ and R⁶ may further contain independently of each other at least one heteroatom selected from O, N, S, and P; or
wherein
(2) R⁵ is a substituted or unsubstituted linear C1-C15 alkyl, branched C3-C15 alkyl, linear C4-C15 alkylcycloalkenyl, or branched C5-C15 alkylcycloalkenyl group; and
wherein R⁶ is a substituted or unsubstituted linear C1-C15 alkylmethoxy, or branched C2-C15 alkylmethoxy group, preferably -CH₂-O-CH₃.

5. The method according to any one of claims 1 to 4, wherein the enone compound of formula (I) is an enone compound of formula (V) or (VI) 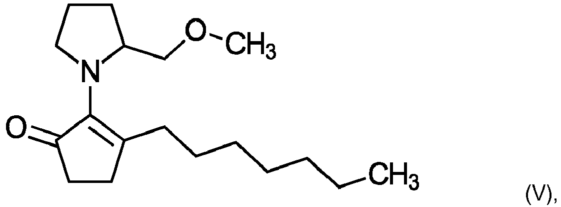 or

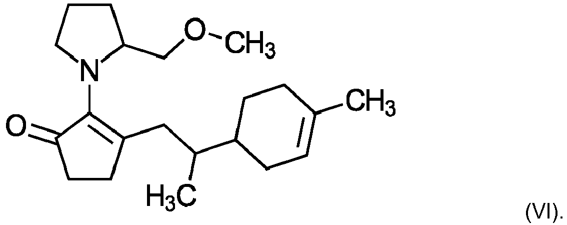

6. The method according to any one of claims 1 to 5, wherein the reaction is conducted in the presence of at least one Brønsted acid or Lewis acid, preferably in the presence of para-toluene sulfonic acid.

7. The method according to any one of claims 1 to 6, wherein the reaction is conducted in the presence of at least one catalyst, preferably in the presence of at least one main group metal based catalyst, transition metal based catalyst, organo-catalyst, or mixtures thereof, more preferably in the presence of at least one transition metal based catalyst, most preferably in the presence of at least one catalyst, which is based on Aluminum (Al), vanadium (V), chrome (Cr), manganese (Mn), iron (Fe), cobalt (Co), nickel (Ni), copper (Cu), zinc (Zn), zirconium (Zr), niobium (Nb), molybdenum (Mo), palladium (Pd), silver (Ag), tungsten (W), iridium (Ir), or platinum (Pt).

8. The method according to any one of claims 1 to 7, wherein the oxidant is any compound, which is able to act as oxygen transfer reagent, preferably oxygen or air, more preferably air, most preferably air at atmospheric pressure.

9. The method according to any one of claims 1 to 8, wherein the at least one catalyst is present in amounts of from 0.001 to 75 mol%, preferably in amounts of from 0.01 to 5 mol%, based on the total amount of starting materials.

10. The method according to any one of claims 1 to 9, wherein the at least one Bronsted acid or Lewis acid is present in amounts of from 0.001 to 75 mol%, preferably in amounts of from 0.01 to 5 mol%, based on the total amount of starting materials.

11. An enone compound of formula (I) obtained by the method according to any one of claims 1 to 10.

12. A washing or cleaning agent comprising the enone compound of formula (I) according to claim 11.

13. An insect repellant composition comprising the enone compound of formula (I) according to claim 11.

14. A pharmaceutical composition comprising the enone compound of formula (I) according to claim 11.

15. Use of the enone compound of formula (I) according to claim 11 as fine chemical, basic chemical, insect repellant, pharmaceutical compound, fragrance or pro-fragrance compound.
